(19) Europäisches Patentamt

European Patent Office

Office européen des brevets

(11) Veröffentlichungsnummer: **0 494 616 A1**

# (12) EUROPÄISCHE PATENTANMELDUNG

(21) Anmeldenummer: **92100094.9**

(22) Anmeldetag: **04.01.92**

(51) Int. Cl.5: **A61K 9/127**, A61K 49/00

(30) Priorität: **07.01.91 CH 21/91**

(43) Veröffentlichungstag der Anmeldung:
**15.07.92 Patentblatt 92/29**

(84) Benannte Vertragsstaaten:
**PT**

(71) Anmelder: **BYK GULDEN LOMBERG CHEMISCHE FABRIK GMBH**
**Postfach 100310, Byk-Guldenstrasse 2**
**W-7750 Konstanz(DE)**

(72) Erfinder: **Beller, Klaus-Dieter**
**Franz-Moser-Strasse 5**
**W-7750 Konstanz 16(DE)**
Erfinder: **Kohl, Bernhard**
**Zum Brühl 9**
**W-7750 Konstanz 18(DE)**
Erfinder: **Linder, Rudolf**
**Felchengang 22**
**W-7750 Konstanz 18(DE)**

(54) **Kontrastmittel für die MR-Diagnostik.**

(57) Es werden wäßrige Kontrastmittel für die MR-Diagnostik enthaltend Eisen(III)-tris-(3,4-dihydroxy-2-methyl-5-pyridylmethylphosphat) in Liposomen beschrieben, die oral und invasal angewendet werden können und sich insbesondere zur Diagnose des Abdominalbereichs und der Nieren eignen.

EP 0 494 616 A1

## Technisches Gebiet

Die Erfindung betrifft neue Kontrastmittel für die MR-Diagnostik.

## Stand der Technik

Es ist bekannt, daß durch Einschluß der in der MR-Diagnostik gebräuchlichen paramagnetischen Metallchelate in Liposomen deren Eigenschaften als MR-Kontrastmittel verändert werden können. Von V. J. Caride et al., Magnetic Resonance Imaging, 2 [1984] 107 - 112, wird berichtet, daß durch Einschluß des Mangan(II)-Chelates mit Diethylentriaminpentaessigsäure (Mn-DTPA) in multilamellaren Liposomen im Vergleich zu freiem Mn-DTPA eine starke Anreicherung in der Leber und vor allem in der Milz und eine Verringerung in den Nieren erreicht wird. Wegen dieser durch die Zubereitung in Liposomen verursachten veränderten Biodistribution werden z.B. in der WO-A-9004943 als MR-Kontrastmittel für die Diagnose von Metastasen in der Leber Liposomen mit einem Durchmesser von weniger als 50 nm mit eingeschlossenen paramagnetischen Substanzen vorgeschlagen.

## Beschreibung der Erfindung

Es wurde nun festgestellt, daß sich mit bestimmten paramagnetischen Chelaten Liposomenzubereitungen herstellen lassen, die sich neben der Erhöhung des MR-Kontrastes im Abdominalbereich vor allem zu einer stark verbesserten Darstellung der Nieren verwenden lassen.

Gegenstand der Erfindung sind daher wäßrige Kontrastmittel für die MR-Diagnostik enthaltend Eisen(III)-tris-(3,4-dihydroxy-2-methyl-5-pyridylmethylphosphat) in Liposomen einer mittleren Teilchengröße von 60 bis 100 nm, vorzugsweise etwa 80 nm.

In vivo-Versuche an Ratten zeigen, daß die erfindungsgemäße Zubereitung bereits bei Verabreichung von 0,01 mMol/kg zu einer hochinformativen Darstellung nicht nur des Nierenbeckens sondern vor allem des Nierengewebes führt. Es sind Details des Nierengewebes, wie z.B. die Markstrahlen darstellbar. Demgegenüber führt die Verabreichung von anderen üblichen paramagnetischen Komplexen nur zu einer anfänglichen Kontrastierung des Nierenbeckens, die mit der zunehmenden Anreicherung der Komplexe im Nierenbecken zu einem "Umkippen" des Kontrastes führt, was die Anwendung von freien paramagnetischen Komplexen für Nierendarstellungen in der Regel nur in sehr begrenztem Umfang als nutzbringend erscheinen läßt.

Weiterhin wurde beobachtet, daß die Verwendung einer erfindungsgemäßen Zubereitung zur Darstellung einer ZNS-Läsion an Ratten zu einem kontrastreicheren Bild führte als es durch Verwendung von paramagnetischen Komplexen gleicher molarer Dosierung erreicht werden kann.

Überraschend vorteilhaft ist die orale Verabreichung von erfindungsgemäßen Zubereitungen zur Darstellung des Gastrointestinalbereichs. So erzeugt eine orale Gabe von 0,02 mMol/kg erfindungsgemäßer Zubereitungen nach wenigen Minuten eine starke Kontrastierung des Magens und des Dünndarms, wobei besonders die klare Strukturierung des Magens ins Auge fällt. Der besondere Nutzen der oralen Verabreichung erfindungsgemäßer Zubereitungen liegt zum einen in der rasch eintretenden Kontrastierung und zum anderen in derem langen Anhalten. Es wird damit möglich, bei Anwendung der bekannten MR-Techniken zur schnellen Erzeugung von Bildern (Snap-Shot-Methoden) dynamische Vorgänge im Gastrointestinalbereich darzustellen.

Zur Herstellung der Liposomen werden die für pharmazeutische Zwecke üblichen amphiphilen Liposomenbildner eingesetzt, die in der Lage sind, unter Ausbildung von Doppelschichten (bilayers) unilamellare oder multilamellare Vesikel zu bilden. Vorzugsweise werden Soja- und Eigelblecithin, gesättigte Phosphatidylcholine, ferner Phosphatidylglycerol, -ethanolamin, -serin und -inositol, Phosphatidsäure, Dicetylphosphat, Cholesterin, Cholesterinester und Stearylester eingesetzt. Dabei dienen die Phospholipide zum Aufbau der Doppelschichten, während Cholesterin und dessen Ester zur Erhöhung der Stabilität der Liposomen eingesetzt werden. Die Phospholipide werden in den für die Liposomenherstellung üblichen Mengen, d.h. in Mengen bis zu 30 % (Gewicht/Volumen), vorzugsweise 5 bis 15 % (Gewicht/Volumen) eingesetzt. Soweit als Hilfsstoffe Cholesterin und dessen Ester zugesetzt werden, geschieht dies in einer Menge von 1 bis 35 Gew.-%, vorzugsweise 1 bis 15 Gew.-%, bezogen auf die Gesamtmenge an Lipid.

Die Herstellung der Liposomen erfolgt nach den in der Literatur beschriebenen Methoden. Beispielsweise werden multilamellare Vesikel (MLV) mit einer Partikelgröße von 100 bis 10000 nm nach der Filmmethode, d.h. Hydratisierung eines Lipidfilms hergestellt. Durch Einwirkung von Ultraschall ausreichender Energie können aus den MLVs kleine unilamellare Vesikel (SUV) einer Partikelgröße von 20 bis 100 nm erzeugt werden. Durch die sogenannten Detergensdialyse, d.h. durch Entfernen von Detergens durch Dialyse, können aus Mischmizellen unilamellare Vesikel einer Partikelgröße von 20 bis 400 nm erhalten werden. Liposomen mit einer Partikelgröße von etwa 50 bis 500 nm können durch die Methode der Phasenumkehrverdampfung (reverse-phase-evaporation), d.h. Abziehen des organischen Lösungsmittels aus einer wäßrigen Emulsion eines in einem organischen Lösungsmittel gelösten Lipidgemisches, gewonnen werden. Weiterhin können Liposomen der gewünschten Partikelgröße nach der French-Press-Methode zubereitet werden, wobei das in einer wäßrigen Phase dispergierte Lipid ein- oder mehrmals durch einen Hochdruckhomogenisator gedrückt wird. Nähere Informationen zur Bereitung der für die vorliegende Erfindung in Frage kommenden Liposomen entnimmt der Fachmann der umfangreichen Fachliteratur. Als besonders hilfreich kann die Offenbarung der WO-A-9004943 herangezogen werden, wo die verschiedenen Methoden zur Herstellung von paramagnetische Teilchen enthaltenden Liposomen mit den entsprechenden Literaturhinweisen ausführlich beschrieben sind.

Die erfindungsgemäßen Zubereitungen können als solche in Ampullen abgefüllt werden oder zu einem löslichen Pulver gefriergetrocknet werden. Die wäßrigen Lösungen werden auf einen pH-Wert mit ausreichender lokaler Gewebeverträglichkeit eingestellt, beispielsweise auf einen pH-Wert von 7 bis 9. Die wäßrigen Lösungen werden oral oder parenteral, insbesondere intravasal verabreicht. Zur MR-Diagnostik beim Menschen werden wäßrige Lösungen verwendet, die den paramagnetischen Komplex in einer Konzentration von 30 bis 500 mMol/Liter enthalten. Pro Anwendung werden ungefähr 1 bis 300 $\mu$Mol des Komplexes pro kg Körpergewicht verabreicht. Für einen Erwachsenen erweist sich für eine i.V.-Anwendung eine Dosis von 1 bis 100 mMol als angebracht.

Herstellungsbeispiele

**1.** Wäßrige Lösung des Eisen(III)-Komplexes von 2-Methyl-3,4-dihydroxy-5-pyridylmethylphosphorsäure

10,6 g (0,045 Mol) 2-Methyl-3,4-dihydroxy-5-pyridylmethylphosphorsäure werden in 2 Äquivalenten 4N Natronlauge als Dinatriumsalz gelöst (pH 7,5). Dazu tropft man innerhalb einer Stunde eine Lösung von 4,05 g (0,015 Mol) Eisen(III)chloridhexahydrat in 20 ml Wasser, wobei der pH-Wert der Lösung durch gleichzeitige Zugabe von einem weiteren Äquivalent 4N Natronlauge auf 7 bis 8 gehalten wird. Man läßt 18 Stunden bei 20°C rühren, füllt mit tridestilliertem Wasser auf 100 ml Volumen auf und filtriert über ein 0,45 $\mu$m Membranfilter. Die resultierende 0,15 molare Lösung der Titelverbindung kann beliebig verdünnt werden.

Das UV-Maximum liegt nach Verdünnung auf $10^{-4}$ Mol/Liter mit 2 x $10^{-3}$-molarem Kaliumphosphatpuffer von pH 7,6 bei 463 nm.

Das IR-Spektrum der zur Trockne eingedampften Komplexlösung in Kaliumbromid zeigt folgende charakteristische Banden:

1475 cm$^{-1}$ (s, C = C und C = N)
1310 cm$^{-1}$ (m, aromatische C-OH)
710 cm$^{-1}$ (n, CH-Deformationsschwingung).

**2.** Eisen(III)-tris-(3,4-dihydroxy-2-methyl-5-pyridylmethylphosphat)

Zu 10 ml (1,5 mMol) einer nach Beispiel 1 hergestellten Lösung wird sukzessive unter Rühren 2N Salzsäure bis pH 2,5 zugetropft. Der ausgefallene Feststoff wird über eine Nutsche filtriert, mit Wasser chloridfrei gewaschen und bei 50°C bis zur Gewichtskonstanz getrocknet. Man erhält 0,8 g (70 % der Theorie) der Titelverbindung als roten feinkristallinen Feststoff vom F. 308 - 310 °C (Zersetzung; gemessen per Fusomat).

**3.** Liposomenzubereitung

**3.1** 10 g Epikuron 200 (Phosphatidylcholin mit einem Molekulargewicht von ca. 760 der Fa Lucas Meyer) werden in 100 ml Ethanol gelöst. Das Lösungsmittel wird am Rotationsverdampfer abgezogen. Es werden 90 ml einer wäßrigen Lösung, enthaltend 10 g Eisen(III)-tris-(3,4-dihydroxy-2-methyl-5-pyridylmethylphosphat) zugegeben. Diese Mischung wird im Wasserbad von ca. 70 - 80 °C eine Stunde belassen. Die Zubereitung wird anschließend im Branson Sonifier Typ 450 beschallt bis eine mittlere Teilchengröße der Liposomen von 80 nm erhalten wird. Alle Manipulationen erfolgen unter Stickstoffbegasung.

**3.2** Es werden 7,5 g Epikuron 200 und 0,1 g Tocopherolacetat in 100 ml Ethanol gelöst. Das Lösungsmittel wird am Rotationsverdampfer abgezogen. Es werden 93 ml einer wäßrigen Lösung, die 10 g Eisen(III)-tris-(3,4-dihydroxy-2-methyl-5-pyridylmethylphosphat) enthält, zugegeben. Zur Hydratisierung wird die Mischung eine Stunde bei 70 - 80°C im Wasserbad belassen. Man beschallt anschließend mit einem Branson Sonifier Typ 450 bis eine mittlere Teilchengröße der Liposomen von 80 nm erhalten wird.

Vergleichsversuch

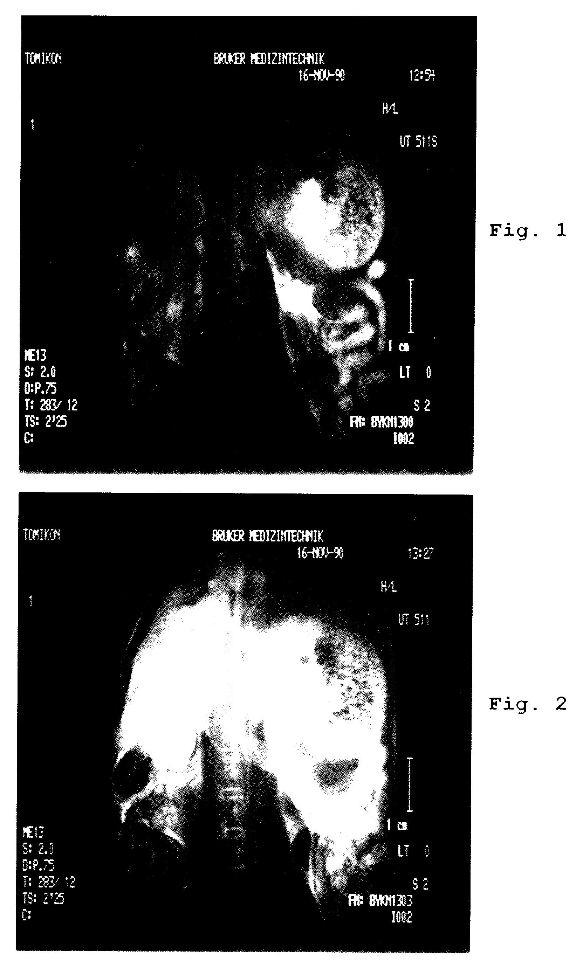

Einer narkotisierten Ratte wurden als erste Dosis 0,05 mMol/kg erfindungsgemäßes MR-Kontrastmittel nach Beispiel 3.1 intravenös verabreicht. Nach 25 Minuten wurden als zweite Dosis weitere 0,1 mMol/kg MR-Kontrastmittel intravenös verabreicht. In Fig. 1 ist eine MR-Aufnahme des Abdomens der Ratte wiedergegeben, die vor Verabreichung der ersten Dosis aufgenommen wurde. Die Abbildung 2 zeigt eine Übersicht über das Abdomen der narkotisierten Ratte 29 Minuten nach Verabreichung der ersten Dosis des MR-Kontrastmittels. Diese Aufnahme illustriert die mit erfindungsgemäßen MR-Kontrastmitteln erreichbare überraschend detailreiche Darstellung sowohl der Organe als auch der Hauptgefäße des Abdomens. Besonders überraschend ist hierbei die räumlich wirkende Wiedergabe der Strukturen des Abdomens.

**Patentansprüche**

1. Wäßrige Kontrastmittel für die MR-Diagnostik enthaltend Eisen(III)-tris-(3,4-dihydroxy-2-methyl-5-pyridylmethylphosphat) in Liposomen.

2. Wäßrige Kontrastmittel nach Anspruch 1, dadurch gekennzeichnet, daß die Liposomen eine mittlere Teilchengröße von 60 bis 100 nm aufweisen.

3. Wäßrige Kontrastmittel nach Anspruch 2, dadurch gekennzeichnet, daß die Liposomen eine mittlere Teilchengröße von etwa 80 nm aufweisen.

4. Verfahren zur Herstellung von wäßrigen Kontrastmitteln für die MR-Diagnostik, dadurch gekennzeichnet, daß man Eisen(III)-tris-(3,4-dihydroxy-2-methyl-5-pyridylmethylphosphat) in Liposomen einarbeitet.

Fig. 1

Fig. 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | US-A-2 497 731 (HOFFMAN)<br>* das ganze Dokument *<br>--- | 1-4 | A61K9/127<br>A61K49/00 |
| D,Y | WO-A-9 004 943 (UNGER ET AL)<br>* Ansprüche 1-15 *<br>--- | 1-4 | |
| P,Y | WO-A-9 112 822 (BYK GULDEN LOMBERG CHEMISCHE FABRIK GMBH)<br>* Ansprüche 1-8 *<br>--- | 1-4 | |
| A | JOURNAL OF THE AMERICAN CHEMICAL SOCIETY Bd. 73, 1951, WASHINGTON D.C.,USA Seiten 3437 - 3439;<br>D.HEYL ET AL: 'PHOSPHATES OF THE VITAMIN B6 GROUP.IV.AN OXIDATION PRODUCT OF CODECARBOXYLASE'<br>* das ganze Dokument *<br>--- | 1-4 | |
| A | EP-A-0 290 047 (SALUTAR,INC.)<br>* Ansprüche 1-13 *<br>----- | 1-4 | |

**RECHERCHIERTE SACHGEBIETE (Int. Cl.5)**

A61K
C07F

Der vorliegende Recherchenbericht wurde für alle Patentansprüche erstellt

| Recherchenort | Abschlußdatum der Recherche | Prüfer |
|---|---|---|
| DEN HAAG | 09 MAERZ 1992 | SITCH W.D.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes Dokument

EPO FORM 1503 03.82 (P0403)